(19) Europäisches Patentamt
European Patent Office
Office européen des brevets

(11) EP 2 661 430 B1

(12) **EUROPEAN PATENT SPECIFICATION**

(45) Date of publication and mention of the grant of the patent:
**14.11.2018 Bulletin 2018/46**

(21) Application number: **11808172.8**

(22) Date of filing: **16.12.2011**

(51) Int Cl.:
***C08K 5/10*** $^{(2006.01)}$ ***C08K 5/1515*** $^{(2006.01)}$
***C09D 7/47*** $^{(2018.01)}$ ***C07D 303/16*** $^{(2006.01)}$

(86) International application number:
**PCT/EP2011/006580**

(87) International publication number:
**WO 2012/084265 (28.06.2012 Gazette 2012/26)**

(54) **GLYCIDYL ESTERS OF ALPHA, ALPHA BRANCHED ACIDS COMPOSITIONS**

GLYCIDYLESTER VON ALPHA, ALPHA-VERZWEIGTEN SÄUREZUSAMMENSETZUNGEN

COMPOSITION D'ESTERS GLYCIDYLIQUES D'ACIDES, ALPHA, ALPHA-RAMIFIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2010 EP 10015959**

(43) Date of publication of application:
**13.11.2013 Bulletin 2013/46**

(73) Proprietor: **Hexion Research Belgium SA**
**1348 Ottignies Louvain-la-Neuve (BE)**

(72) Inventors:
- **STEINBRECHER, Christophe**
**B-1348 Ottignies Louvain-la-Neuve (BE)**
- **LE FEVERE DE TEN HOVE, Cédric**
**B-1348 Ottignies Louvain-la-Neuve (BE)**
- **VAN'T SAND, Robert**
**B-1348 Ottignies Louvain-la-Neuve (BE)**
- **HEYMANS, Denis**
**B-1348 Ottignies Louvain-la-Neuve (BE)**
- **KOTLEWSKA, Aleksandra**
**B-1348 Ottignies Louvain-la-Neuve (BE)**

(56) References cited:
**EP-A1- 1 283 226**
**WO-A1-96/33245**
**WO-A1-99/51659**
**JP-A- 4 314 797**
**JP-A- 60 032 803**
**US-B1- 6 342 615**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 661 430 B1

## Description

The present invention relates to a composition of α,α-branched alkane carboxylic acids glycidyl esters with a defined isomeric composition; which can lead for example to improved leveling of the coatings derived thereof.

More in particular the invention relates to the compositions of aliphatic tertiary saturated carboxylic acids or α,α-branched alkane carboxylic acids, which contain 9 or 13 carbon atoms and which provide glycidyl esters with a branching level of the alkyl groups depending on the olefin feedstock used and/or the oligomerisation process thereof, and which is defined as below.

It is generally known from e.g. US 2,831,877, US 2,876,241, US 3,053,869, US 2,967,873 and US 3,061,621 that mixtures of α,α-branched alkane carboxylic acids can be produced, starting from mono-olefins, carbon monoxide and water, in the presence of a strong acid.
One of the more recent method has been disclosed in EP 1033360A1. The problem of providing better softening derivatives of α,α-branched acids, manufactured from alkenes, carbon monoxide and water and a nickel catalyst was solved therein by a process, which actually comprised:

(a) oligomerisation of butene;

(b) separation of butene dimers and/or trimers from the oligomerisate;

(c) conversion of the butene dimers and/or trimers into carboxylic acids;

(d) conversion of the carboxylic acids into the corresponding vinyl esters showing attractive softening properties when mixed into other polymers or if used as comonomers in coatings.

If the olefin feed is based on Raf. II or Raf III or any mixture rich in n-butene isomers on the total olefins, the subsequently mixture of neo-acid (C9 or C13 acids) derivatives will provide a mixture where the sum of the concentration of the blocked isomers and the concentration of the highly branched isomers is maximum 55%, preferably below 40%, and most preferably below 30%.

The glycidyl esters can be obtained according to PCT/EP2010/003334 or the US6433217.

WO 9951659 is about the use of glycidyl esters of branched acid to produce low viscosity polyester resins by reaction of the epoxy functionality and the acid group of the polyester resins. EP 1283226 A1 is another example of the use of glycidyl ester of branched acid in modification of acrylic resins. JP 4314797 A is about the use of branched glycidyl esters and polyalcohol ester blends in mineral or synthetic oil. WO 9633245 describe the use of branched glycidyl ester in resins. JP 60032803 A is about the use of glycidyl ester to de-functionalize hydroxystyrene polymer as agent to treat metal surfaces. US 6342615 B1 is about the purification of a glycidyl ester by a vacuum thin film distillation, which provide a resin with higher glass transition temperature.

We have discovered that well chosen blend of isomers of the glycidyl ester of mixture compositions of neo-acid (C9 or C13 acids) glycidyl ester, is providing for example a good leveling of a coating, is a mixture where the sum of the concentration of the blocked isomers and the concentration of the highly branched isomers is maximum 55%, preferably below 40%, and most preferably below 30% weight on total composition.

We have further discovered that well chosen blend of isomers of the glycidyl ester of, for example, neononanoic acids give different and unexpected performance in combination with some particular polymers such as polyester polyols.

The isomers are described in Table 1 and illustrated in Figure 1.

We have found that the performance of the glycidyl ester compositions derived from the branched acid is depending on the branching level of the alkyl groups $R^1$, $R^2$ and $R^3$, for example the neononanoic acid has 3, 4 or 5 methyl groups. Highly branched isomers are defined as isomers of neo-acids having at least 5 methyl groups.

Mixture compositions of neononanoic (C9) acids glycidyl esters providing for example a good leveling of a coating, is a mixture where the sum of the concentration of the blocked isomers and the concentration of the highly branched isomers is maximum 55%, preferably below 40%, and most preferably below 30% weight on total composition.

Furthermore the above compositions of neononanoic acids glycidyl esters mixture is comprising 2,2-dimethyl heptanoic acid glycidyl ester and 2-methyl 2-ethyl hexanoic acid glycidyl ester and 2-methyl 2 ethyl 3-methyl pentanoic acid glycidyl esters.

The above compositions of the glycidyl ester mixture is comprising 2,2-dimethyl heptanoic acid glycidyl ester in 4 to 10 weight% and 2-methyl 2-ethyl hexanoic acid glycidyl ester in 40 to 70 weight% and 2-methyl 2-ethyl 3-methyl pentanoic acid glycidyl esters (the sum of the stereoisomers) in 10 to 25 weight% on total composition.
A preferred composition is comprising a mixture of 2,2-dimethyl heptanoic acid glycidyl ester in 5 to 10 weight% and 2-methyl 2-ethyl hexanoic acid glycidyl ester in 45 to 65 weight% and 2-methyl 2-ethyl 3-methyl pentanoic acid glycidyl esters (the sum of the stereoisomers) in 12 to 22 weight% on total composition.

A further preferred composition is comprising a mixture of 2,2-dimethyl heptanoic acid glycidyl ester in 6 to 9 weight% and 2 methyl 2 ethyl hexanoic acid glycidyl ester in 47 to 61 weight% and 2-methyl 2-ethyl 3-methyl pentanoic acid glycidyl esters (the sum of the stereoisomers) in 14 to 21 weight% on total composition.

The above glycidyl esters compositions can be used for example, as reactive diluent or as momomer in binder compositions for paints or adhesives.

The glycidyl esters compositions can be used as reactive diluent for epoxy based formulations such as exemplified in the technical brochure of Hexion(Product Bulletin: Cardura N10 The Unique Reactive Diluent).

Other uses of the glycidyl ester are the combinations with polyester polyols, or acrylic polyols, or polyether polyols. The combination with polyester polyols such as the one used in the car industry coating leads to coating system with attractive coating appearance.

**Methods used**

The isomer distribution of neo-acid can be determined using gas chromatography, using a flame ionization detector (FID). 0.5 ml sample is diluted in analytical grade dichloromethane and n-octanol may be used as internal standard. The conditions presented below result in the approximate retention times given in Table 1. In that case n-octanol has a retention time of approximately 8.21 minute.

The GC method has the following settings:

Column: CP Wax 58 CB (FFAP), 50 m x 0.25 mm, df = 0.2 μm
Oven program : 150°C (1.5 min) - 3.5°C/min - 250°C (5 min) = 35 min
Carrier gas: Helium
Flow : 2.0 mL/min constant
Split flow : 150 mL/min
Split ratio: 1:75
Injector temp : 250°C
Detector temp : 325°C
Injection volume: 1 μL

CP Wax 58 CB is a Gas chromatography column available from Agilent Technologies.

The isomers of neononanoic acid as illustrative example have the structure ($R^1$ $R^2$ $R^3$)-C-COOH where the three R groups are linear or branched alkyl groups having together a total of 7 carbon atoms.

The structures and the retention time, using the above method, of all theoretical possible neononanoic isomers are drawn in Figure 1 and listed in Table 1.

The isomers content is calculated from the relative peak area of the chromatogram obtained assuming that the response factors of all isomers are the same.

**Table 1:** Structure of all possible neononanoic isomers The isomer distribution of glycidyl esters of neo-acid can be determined by gas chromatography, using a flame ionization detector (FID). 0.5 ml sample is diluted in analytical grade dichloromethane.

| | R1 | R2 | R3 | Methyl groups | Blocking | Retention time [Minutes] |
|---|---|---|---|---|---|---|
| V901 | Methyl | Methyl | n-pentyl | 3 | No | 8.90 |
| V902 | Methyl | Methyl | 2-pentyl | 4 | Yes | 9.18 |
| V903 | Methyl | Methyl | 2-methyl butyl | 4 | No | 8.6 |
| V904 | Methyl | Methyl | 3-methyl butyl 1,1-dimethyl | 4 | No | 8.08 |
| V905 | Methyl | Methyl | propyl 1,2-dimethy | 5 | Yes | 10.21 |
| V906 | Methyl | Methyl | propyl 2,2-dimethyl | 5 | Yes | 9.57 |
| V907 | Methyl | Methyl | propyl | 5 | No | 8.26 |
| V908 | Methyl | Methyl | 3-pentyl | 4 | Yes | 9.45 |
| V909 | Methyl | Ethyl | n-butyl | 3 | No | 9.28 |
| V910 K1 | Methyl | Ethyl | s-butyl | 4 | Yes | 9.74 |
| V910 K2 | Methyl | Ethyl | s-butyl | 4 | Yes | 9.84 |

(continued)

| | R1 | R2 | R3 | Methyl groups | Blocking | Retention time [Minutes] |
|---|---|---|---|---|---|---|
| V911 | Methyl | Ethyl | i-butyl | 4 | No | 8.71 |
| V912 | Methyl | Ethyl | t-butyl | 5 | Yes | 9.64 |
| V913 | Methyl | n-propyl | n-propyl | 3 | No | 8.96 |
| V914 | Methyl | n-propyl | i-propyl | 4 | Yes | 9.30 |
| V915 | Methyl | i-propyl | i-propyl | 5 | Yes | 9.74 |
| V916 | Ethyl | Ethyl | n-propyl | 3 | No | 9.44 |
| V917 | Ethyl | Ethyl | i-propyl | 4 | Yes | 10.00 |

The GC method has the following settings:

Column: CP Wax 58 CB (FFAP), 50 m x 0.2 mm, df = 0.52 μm

Oven : 175°C (5 min)- 1°C/min - 190°C (0 min) - 10°C/min - 275°C (11.5 min)

Flow : 2.0 mL/min, constant flow

Carrier gas: Helium

Split ratio: 1:75

Injection volume:1 μL

S/SL injector:250°C

CP Wax 58 CB is a Gas chromatography column available from Agilent Technologies.

The isomers of glycidyl esters of neononanoic acid as illustrative example have the structure ($R^1$ $R^2$ $R^3$)-C-COO-$CH_2$-CH(O)$CH_2$ where the three R groups are linear or branched alkyl groups having together a total of 7 carbon atoms.

The isomers content is calculated from the relative peak area of the chromatogram obtained assuming that the response factors of all isomers are the same.

GC-MS method can be used to identify the various isomers providing that the analysis is done by a skilled analytical expert.

**Figure 1:** Structure of all possible neononanoic isomers

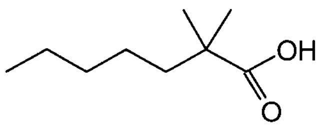

V901 = E

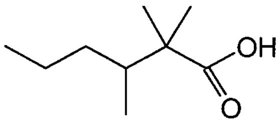

V902 = F

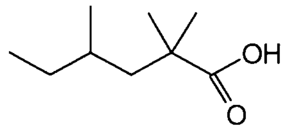

V903 = G

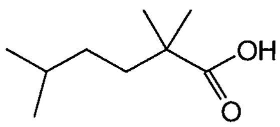

V904 = H

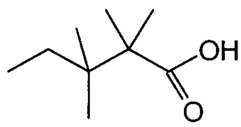

V905 = C

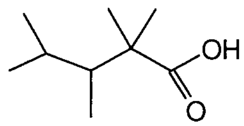

V906 = D

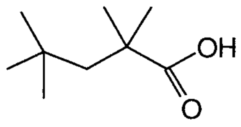

V907 = A

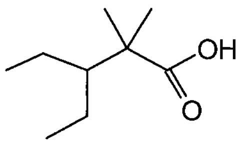

V908 = I

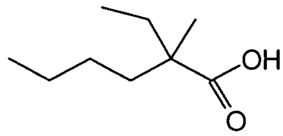

V909 = J

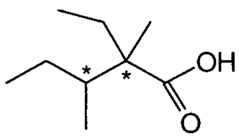

V910** = K1

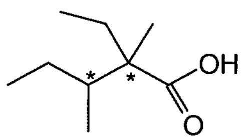

V910** = K2

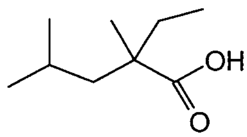

V911 = L

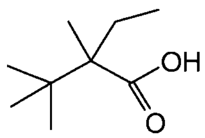

V912 = B2

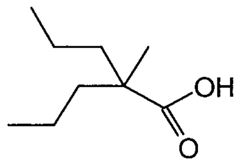

V913 = M

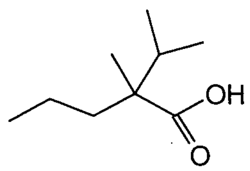

V914 = P

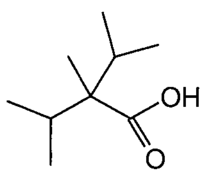

V915 = B1

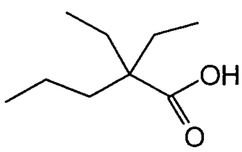

V916 = Q

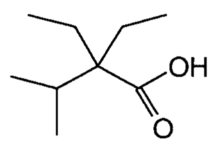

V917 = R

**Test methods for the characterization of the resins**

The molecular weights of the resins are measured with gel permeation chromatography (Perkin Elmer/ Water) in THF solution using polystyrene standards. Viscosity of the resins are measured with Brookfield viscometer (LVDV-I) at indicated temperature. Solids content are calculated with a function (Ww-Wd) / Ww × 100%. Here Ww is the weight of a wet sample, Wd is the weight of the sample after dried in an oven at a temperature 110 °C for 1 hour.

Tg (glass transition temperature) has been determined either with a DSC 7 from Perkin Elmer or with an apparatus from TA Instruments Thermal Analysis. Scan rates were respectively 20 and 10°C/min. Only data obtained

in the same experimental conditions have been compared. If not, the temperature difference occurring from the different scanning rate has been proved not significant for the results compared.

**Methods for the Characterization of the Clear Coats**

Pot-life

Pot-life is determined by observing the elapsed time for doubling of the initial viscosity at room temperature, usually 24.0 ± 0.5 °C. The initial viscosity of the clear coat is defined at 44-46 mPa.s for Part 1 and 93-108 mPa.s for Part 3 measured with Brookfield viscometer.

Application of clearcoat

Q-panels are used as substrates. Then the panels are cleaned by a fast evaporating solvent methyl ethyl ketone or acetone. For Part 1 the clearcoat is spray-applied on Q-panels covered with basecoat; for Parts 2 & 3 the clearcoat is barcoated directly on Q-panels.

Dust free time

The dust free time (DFT) of clear coat is evaluated by vertically dropping a cotton wool ball on a flat substrate from a defined distance. When the cotton ball contacts with the substrate, the substrate is immediately turned over. The dust free time is defined as the time interval at which the cotton wool ball no longer adhered to the substrate.

Hardness development

Hardness development is followed using pendulum hardness tester with Koenig method.

**Blocking isomers**

Whereas the carbon atom in alpha position of the carboxylic acid is always a tertiary carbon atom, the carbon atom(s) in

β position can either be primary, secondary or tertiary. Neononanoic acids (V9) with a secondary or a tertiary carbon atoms in the β position are defined as blocking isomers (Figures 2 and 3).

Fig 2

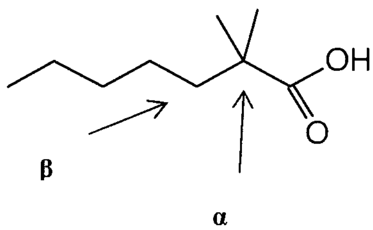

Fig 3

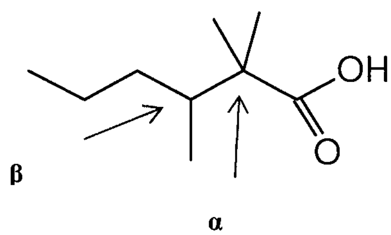

**Figure 2:** Example of a Non-blocked V9 Structure

**Figure 3:** Example of a Blocked V9 Structure

The use of the glycidyl esters compositions, discussed here above, is as reactive diluent or as momomer in binder compositions for paints and adhesives.

These uses can be based on a polyester polyol resin comprising the above composition glycidyl ester and/or an acrylic polyol resin comprising the above composition glycidyl ester and/or a polyether polyol resin comprising the above composition glycidyl ester and/or an epoxy resin formulation comprising the above composition glycidyl ester.

## Examples

### Chemicals used

- **Cardura™ E10:** available from Momentive Specialty Chemicals
- Neononanoic glycidyl ester from Momentive Specialty Chemicals
- **GE9S:** neononanoic glycidyl ester of composition A (see table 2 below)
- **GE9H:** neononanoic glycidyl ester of composition B (see table 2 below)
- **Neononanoic glycidyl ester of composition C** (see table 2 below)
- **Neononanoic glycidyl ester of composition D** (see table 2 below)
- **Neononanoic glycidyl ester of composition E** (see table 2 below) **GE5:** glycidyl ester of pivalic acid obtained by reaction of the acid with epichlorhydrin.
- **Ethylene glycol** from Aldrich
- **Monopentaerythritol** : available from Sigma - Aldrich
- **Methylhexahydrophtalic anhydride:** available from Sigma - Aldrich
- **Boron trifluoride diethyl etherate** ($BF_3 \cdot OEt_2$) from Aldrich
- **Acrylic acid :** available from Sigma - Aldrich
- **Hydroxyethyl methacrylate:** available from Sigma - Aldrich
- **Styrene** : available from Sigma - Aldrich
- **2-Ethylhexyl acrylate** : available from Sigma - Aldrich
- **Methyl methacrylate** : available from Sigma - Aldrich
- **Butyl acrylate** : available from Sigma - Aldrich
- **Xylene**
- **Di-t-Amyl Peroxide** is Luperox DTA from Arkema
- **tert-Butyl peroxy-3,5,5-trimethylhexanoate:** available from Akzo Nobel
- **n-Butyl Acetate** from Aldrich
- **Dichloromethane** from Biosolve
- **Thinner: A:** is a mixture of Xylene 50wt%, Toluene 30wt%, ShellsolA 10wt%, 2-Ethoxyethylacetate 10wt%. **Thinner B:** is butyl acetate
- **Curing agents, HDI:** 1,6-hexamethylene diisocyanate trimer, Desmodur N3390 BA from Bayer Material Science or Tolonate HDT LV2 from Perstorp
- **Leveling agent:** 'BYK 10 wt%' which is BYK-331 diluted at 10% in butyl acetate
- **Catalyst:** 'DBTDL 1 wt%' which is Dibutyl Tin Dilaurate diluted at 1wt% in butyl acetate

**Table 2:** Composition of the neononanoic glycidyl ester (according to the described gas chromatography method for glycidyl esters of neo-acid)

| Glycidyl ester of acid V9XX (described in Table 1) | A (%) | B (%) | C (%) | D (%) | E (%) |
|---|---|---|---|---|---|
| V901 | 6.5 | 0.1 | 3.7 | 0.1 | 8.9 |
| V902 | 0.6 | 2.55 | 0.6 | 2.4 | 0.7 |
| V903 | 1.1 | 0.7 | 0.3 | 1.0 | 2.0 |
| V904 | 0.8 | 1 | 0.1 | 2.2 | 1.8 |
| V905 | 0.2 | 13.1 | 0.5 | 4.1 | 0.1 |
| V906 | 0.4 | 11.6 | 0.4 | 9.6 | 0.4 |
| V907 | 0.2 | 15.4 | 0.1 | 36.4 | 0.6 |
| V908 | 0.1 | 0 | 0.1 | 0.0 | 0.1 |
| V909 | 54.8 | 2.55 | 52.8 | 2.4 | 52.8 |
| V910 K1 | 7.8 | 0 | 10.0 | 0.0 | 6.5 |
| V910 K2 | 7.7 | 0.6 | 12.8 | 0.4 | 4.8 |

(continued)

| Glycidyl ester of acid V9XX (described in Table 1) | A (%) | B (%) | C (%) | D (%) | E (%) |
|---|---|---|---|---|---|
| V911 | 2.4 | 1.2 | 0.7 | 2.0 | 4.2 |
| V912 | 0.0 | 28.3 | 0.0 | 22.4 | 0.0 |
| V913 | 6.8 | 0.1 | 6.4 | 0.1 | 6.5 |
| V914 | 4.5 | 0 | 3.8 | 0.0 | 5.7 |
| V915 | 0.6 | 22.3 | 0.6 | 16.8 | 0.4 |
| V916 | 4.4 | 0.1 | 5.2 | 0.1 | 3.8 |
| V917 | 1.1 | 0.4 | 2.1 | 0.1 | 0.5 |

**Example 1**

The following constituents were charged to a reaction vessel equipped with a stirrer, a condenser and a thermometer: 92.4 grams of GE9S, 24.0 grams of Butyl Acetate. That initial reactor charge has been heated up to 135°C. Then, the following mixture was added over a period of 1h20 while keeping the temperature constant: 30.7 grams of acrylic acid, 1.2 grams of Di-t-Amyl Peroxide, 12.0 grams of n-Butyl Acetate. After further adding 1.2 grams of Di-t-Amyl Peroxide and 20.4 grams of n-Butyl Acetate, a post-cooking was pursued at 135°C for 1 h. The acrylic polyol had a molecular weight (Mw) of 11400 Daltons and a Tg of about -10°C.

**Example 2 Comparative**

The following constituents were charged to a reaction vessel equipped with a stirrer, a condenser and a thermometer: 92.4 grams of GE9H, 24.0 grams of Butyl Acetate. That initial reactor charge has been heated up to 135°C. Then, the following mixture was added over a period of 1h18 while keeping the temperature constant: 30.2 grams of acrylic acid, 1.2 grams of Di-t-Amyl Peroxide, 12.0 grams of n-Butyl Acetate. After further adding 1.2 grams of Di-t-Amyl Peroxide and 20.4 grams of n-Butyl Acetate, a post-cooking was pursued at 135°C for 1h. The acrylic polyol had a molecular weight (Mw) of 8600 Daltons and a Tg of about +26°C.

Observations: Tg of acrylic polyols is impacted by the composition of the neononanoic glycidyl ester (see examples 1, 2) .

**Example 3**

The adducts of Glycidyl neononanoate, GE9S and acrylic acid or methacrylic acid

The adducts of Glycidyl neononanoate GE9S (see table 3) with acrylic acid (ACE-adduct) and with methacrylic acid (MACE-adduct) are acrylic monomers that can be used to formulate hydroxyl functional (meth)acrylic polymers.

**Table 3:** Compositions of the adducts intakes in parts by weight

| | Acrylic acid adduct | Meth acrylic acid adduct |
|---|---|---|
| Initial reactor charge | | |
| GE9S | 250 | 250 |
| Acrylic acid | 80 | |
| Methacrylic acid | | 96.5 |
| Radical Inhibitor | | |
| 4-Methoxy phenol | 0.463 | 0.463 |
| Catalyst | | |
| DABCO T9 (0.07 wt% on Glycidyl ester) | 0.175 | 0.175 |

- DABCO T9 and 4-Methoxy phenol (185 ppm calculated on glycidyl ester weight), are charged to the reactor.
- The reaction is performed under air flow (in order to recycle the radical inhibitor).

- The reactor charge is heated slowly under constant stirring to about 80°C, where an exothermic reaction starts, increasing the temperature to about 100°C.

- The temperature of 100°C is maintained, until an Epoxy Group Content below 30 meq/kg is reached. The reaction mixture is cooled to room temperature.

**Example 4**

Acrylic resins for high solids automotive refinish clearcoats A glass reactor equipped with stirrer was flushed with nitrogen, and the initial reactor charge (see table 4) heated to 160°C. The monomer mixture including the initiator was then gradually added to the reactor via a pump over 4 hours at this temperature. Additional initiator was then fed into the reactor during another period of 1 hour at 160°C. Finally the polymer is cooled down to 135°C and diluted to a solids content of about 68% with xylene.

**Table 4:** Acrylic resins recipe

| Initial Reactor Charge | Weight % | in Reactor 1L (g) |
|---|---|---|
| GE9S (or GE9H comparative) | 28.2 | 169.1 |
| Xylene | 2.7 | 16.2 |
| Feeding materials | Weight % | in Reactor 1L (g) |
| Acrylic acid | 10 | 59.8 |
| Hydroxy ethyl methacrylate | 16.0 | 96.0 |
| Styrene | 30.0 | 180.0 |
| Methyl methacrylate | 15.8 | 95.0 |
| Di t-Amyl peroxide | 4.0 | 24.0 |
| Xylene | 8.3 | 49.8 |
| Post cooking | Weight % | in Reactor 1L (g) |
| Di t-Amyl peroxide | 1.0 | 6.0 |
| Xylene | 3.0 | 18.0 |
| Solvent adding at 130°C | Weight % | in Reactor 1L (g) |
| Xylene | 50.8 | 305.0 |
| | | |
| Final solids content | 61.8% | |
| Hydroxyl content | 4.12% | |

**Example 5**

Clear coats for automotive refinish

Solvents were blended to yield a thinner mixture of the following composition (table 5):

**Table 5:** Thinner composition

| Thinner | Weight % in solvent blend, theory |
|---|---|
| Toluene | 30.1% |
| ShellSolA | 34.9% |
| 2-ethoxyethyl acetate | 10.0% |
| n-Butyl acetate | 25.0% |

(continued)

| Thinner | Weight % in solvent blend, theory |
|---|---|
| **Total** | **100%** |

A clearcoat was then formulated (table 6) with the following ingredients (parts by weight):

**Table 6:** Clearcoat formulation

| Resin of example ex 4 | Desmodur N3390 | BYK 10wt% in ButAc | DBTDL 1wt% in ButAc | Thinner |
|---|---|---|---|---|
| 80.1 | 27.01 | 0.53 | 1.17 | 40.45 |

| **Clearcoat properties** | **GE9H** (comparative) | **GE9S** |
|---|---|---|
| Volatile organic content | 480 g/l | 481 g/l |
| Initial viscosity | 54 cP | 54 cP |
| Dust free time | 12 minutes | 14.5 minutes |
| Koenig Hardness after 6 h | 8.3 s | 7.1s |

**Example 6**

Acrylic resins for first finish automotive topcoats
GE9S based (28%) acrylic polymers for medium solids first-finish clear coats
A reactor for acrylic polyols is flushed with nitrogen and the initial reactor charge (see table 7) heated to 140°C. At this temperature the monomer mixture including the initiator is added over 4 hours to the reactor via a pump. Additional initiator is fed into the reactor during one hour, and then the mixture is kept at 140°C to complete the conversion in a post reaction. Finally the polymer is cooled down and diluted with butyl acetate to a solids content of about 60%.

**Table 7:** Acrylic resins recipe

| | Intakes (parts by weight) |
|---|---|
| **Initial reactor charge** | |
| GE9S | 164.40 |
| Xylene | 147.84 |
| **Monomer mixture** | |
| Acrylic acid | 53.11 |
| Butyl methacrylate | 76.88 |
| Butyl acrylate | 48.82 |
| Hydroxy-ethyl methacrylate | 27.20 |
| Styrene | 177.41 |
| Methyl methacrylate | 47.31 |
| **Initiator** | |
| Di-*tert*.-amyl peroxide (DTAP) | 8.87 |
| **Post addition** | |
| Di-*tert*.-amyl peroxide | 5.91 |
| **Solvent** (to dilute to 60% solids) | |
| Butyl acetate | 246.00 |
| Total | 1000.0 |

**Clear lacquer Formulation**

Clear lacquers are formulated (see table 8) from the acrylic polymers by addition of Cymel 1158 (curing agent

from CYTEC), and solvent to dilute to spray viscosity. The acidity of the polymer is sufficient to catalyze the curing process, therefore no additional acid catalyst is added. The lacquer is stirred well to obtain a homogeneous composition.

**Table 8:** Clear lacquer formulations and properties of the polymers

| | Intakes (part by weight) |
|---|---|
| Ingredients | |
| Acrylic polymer | 60.0 |
| Cymel 1158 | 8.8 |
| Butyl acetate (to application viscosity) Properties | 24.1 |
| Solids content [% m/m] | 45.3 |
| Density [g/ml] | 0.97 |
| VOC [g/l] | 531 |

**Application and cure**

The coatings are applied with a barcoater on Q-panels to achieve a dry film thickness of about 40μm. The systems are flashed-off at room temperature for 15 minutes, then baked at 140°C for 30 minutes. Tests on the cured systems are carried out after 1 day at 23°C.

**Example 7**

In a reactor equipped with an anchor stirrer, a thermometer, condenser and monomer/initiator feeding system, 188.6g of GE9S and 90g of ethoxypropanol (EPR) were loaded and heated to about 150°C (see table 9). A mixture of 52g of hydroxyethylmethacrylate (HEMA), 160g of styrene, 68g of acrylic acid (AA), 10g of dicumylperoxide (DCP), 37.7g of GE9S and 40g of ethoxypropanol (EPR) were added over 2 hours 30 minutes to the reactor while keeping its content at 150°C. After the feed, the reactor content was held for 30 minutes at this temperature. After the 30 minutes hold period, 108g of HEMA, 30g of AA, 142g of isobutyl methacrylate (IBMA), 5g of DCP and 45 grams of EPR were added over 2 hours and 30 minutes at about 150°C followed by a rinsing step for the feed system with 5 g of EPR. After the rinsing step, the content of the reactor was held for 2 hours at 150°C. The reactor content was cooled down to 100°C and 100 parts of EPR were distilled off at atmospheric pressure.

The polyacrylate polyol has a solids content of the solution of 90% by weight.

**Table 9:** Composition of polyol

| | Materials | Intake (g) |
|---|---|---|
| **Initial charge** | | |
| | EPR | 90 |
| | GE9S | 188.6 |
| **Monomer Addition 1** | | |
| | AA | 68 |
| | Styrene | 160 |
| | GE9S | 37.7 |
| | HEMA | 52 |
| | EPR | 40 |
| | DCP | 10 |
| **Monomer Addition 2** | | |
| | AA | 30 |
| | IBMA | 142 |
| | HEMA | 108 |
| | DCP | 5 |
| | EPR | 45 |
| **TOTAL** | | 976.3 |

**Example 8**

The following constituents were charged to a reaction vessel : 0.7153 grams of a neononanoic glycidyl ester of composition C, 0.5958 grams of hexahydro-4-methylphthalic anhydride, 0.0014 grams of ethylene glycol. The reaction took place for 3 to 4 days at 140°C. The sample has been dried by evaporation. The polyester had a molecular weight (Mn) of 4700 Daltons and a Tg of +18.8°C.

**Example 9 comparative**

The following constituents were charged to a reaction vessel : 0.5823 grams of a neononanoic glycidyl ester of composition D, 0.4775 grams of hexahydro-4-methylphthalic anhydride, 0.0011 grams of ethylene glycol, 0.2841 grams of n-Butyl Acetate. The reaction took place for 3 to 4 days at 120 - 140°C and the solvent was then thoroughly removed by evaporation. The polyester had a molecular weight (Mn) of 5000 Daltons and a Tg of +43.7°C.

**Example 10**

The following constituents were charged to a reaction vessel : 0.7235 grams of a neononanoic glycidyl ester of composition E, 0.5981 grams of hexahydro-4-methylphthalic anhydride, 0.0014 grams of ethylene glycol. The reaction took place for 3 to 4 days at 140°C. The sample has been dried by evaporation. The polyester had a molecular weight (Mn) of 5700 Daltons and a Tg of +17.6°C.

Observations: Tg of polyesters is impacted by the composition of the neononanoic glycidyl ester (see examples 8, 9, 10).

The resins of the examples can be formulated in coating compositions such as 2K (polyurethane) with a low VOC (volatile organic compound) level and still providing and excellent appearance.

**Example 11**

Monopentaerythritol / Methylhexahydrophtalic anhydride / GE9S (1/3/3 molar ratio) = CE-GE9S

80.4 g amount of butylacetate, 68.3 g of monopentaerythritol, 258.2 g of methylhexahydrophthalic anhydride are loaded in a glass reactor and heated to reflux until complete dissolution. Afterwards, the temperature is decreased down to 120°C and 333.0 g of GE9S are added over about one hour. The cooking is pursued at 120°C for the time needed to decrease epoxy group content and acid value down to an acid value below 15 mg KOH/g. Then, further 82.4 g of butylacetate are added. Test results are indicated in table 10.

**Example 12 Comparative**

Monopentaerythritol / Methylhexahydrophtalic anhydride / GE9H (1/3/3 molar ratio) = CE-GE9Ha

80.4 g amount of butylacetate, 68.3 g of monopentaerythritol, 258.2 g of methylhexahydrophthalic anhydride are loaded in a glass reactor and heated to reflux until complete dissolution. Afterwards, the temperature is decreased down to 120°C and 337.1 g of GE9H are added over about one hour. The cooking is pursued at 120°C for the time needed to decrease epoxy group content and acid value down to an acid value below 15 mg KOH/g. Then, further 83.4 g of butylacetate are added. Test results are indicated in table 10.

**Table 10:** Polyesters characterization

| Polyester resin | SC (%) | Mw (Da) | Mn (Da) | Mw/Mn (PDI) | Viscosity (cP) |
|---|---|---|---|---|---|
| CE-GE9S | 78.6 | 974 | 919 | 1.06 | 2450 |

(continued)

| Polyester resin | SC (%) | Mw (Da) | Mn (Da) | Mw/Mn (PDI) | Viscosity (cP) |
|---|---|---|---|---|---|
| CE-GE9Ha | 80.0 | 921 | 877 | 1.05 | 6220 |
| SC : solids content | | | | | |

**Formulation of the Clear Coats**

The clearcoat has been formulated as follows: CE-GEx polyester with Tolonate HDT LV2 as hardener (0.03 wt% DBTDL) (see table 11).

**Table 11:** Clear coats, formulations

| CE-GEx | Binder 2 (g) | HDI (g) | BYK 10wt% (g) | DBTDL 1wt% (g) | Thinner B (g) |
|---|---|---|---|---|---|
| **GE9S** | 80.0 | 36.56 | 0.72 | 3.15 | 89.75 |
| **GE9Ha** | 80.4 | 37.27 | 0.73 | 3.20 | 87.83 |

**Characterization of the Clear Coats**

The clearcoat formulations are barcoat applied on degreased Q-panel. The panels are dried at room temperature, optionally with a preliminary stoving at 60°C for 30 min. Results are indicated in table 12.

**Table 12:** Clear coats, performances

| CE-GEx | SC (%) | Drying conditions | **DFT** (min) *Cotton Balls* | **Koenig Hardness** (s) | | |
|---|---|---|---|---|---|---|
| | | | | **6h** | **24h** | **7d** |
| GE9S | 48.4 | RT | 223 | 3 | 17 | 159 |
| GE9Ha | 49.2 | RT | 91 | 3 | 36 | 212 |
| | | | | | | |
| GE9S | 48.4 | Stoving 30 min/60°C | Dust free out of oven | 4 | 44 | 174 |
| GE9Ha | 49.2 | Stoving 30 min/60°C | Dust free out of oven | 10 | 55 | 211 |

**Example 13 comparative**

The following constituents were charged to a reaction vessel : 2.5500 grams of a neononanoic glycidyl ester of composition D, 1.1571 grams of dichloromethane, 0.0137 grams of boron trifluoride diethyl etherate. The reaction took place for 3 days at room temperature and the solvent was then thoroughly removed by evaporation. The polyether had a molecular weight (Mw) of 1900 Daltons and a Tg of -40.5°C.

**Example 14**

The following constituents were charged to a reaction vessel : 2.5438 grams of a neononanoic glycidyl ester of composition C, 1.0150 grams of dichloromethane, 0.0128 grams of boron trifluoride diethyl etherate. The reaction took place for 3 days at room temperature and the solvent was then thoroughly removed by evaporation. The polyether had a molecular weight (Mw) of 1500 Daltons and a Tg of -51.1°C.

Observations: Tg of the modified polyether resin is impacted by the composition of the neononanoic glycidyl ester (see examples 13, 14).

**Example 15**

Polyether resin

The following constituents were charged to a reaction vessel equipped with a stirrer, a thermometer and a

condenser: 134 grams of di-Trimethylol propane (DTMP), 900 grams of glycidyl neononanoate, GE9S, 135.5 grams of n-butylacetate (BAC) and 2.5 grams of Tin 2 Octoate. The mixture was heated to its reflux temperature of about 180° C. for about 4 hours till the glycidyl neononaoate was converted to an epoxy group content of less than 0.12 mg/g. After cooling down the polyether had a solids content of about 88%.

**Example 16 Comparative**

Polyether resin

The following constituents were charged to a reaction vessel equipped with a stirrer, a thermometer and a condenser: 28.8 grams of monopentaerythritol, 201.5 grams of Cardura E10P, 19.4 grams of n-butylacetate and 0.3552 grams of Tin (II) 2-ethylhexanoate. The mixture was heated to a temperature of about 180° C for about 6 hours till the Cardura E10P was converted to an epoxy group content of about 25 mmol/kg. After cooling down the polyether had a solids content of about 94%.

**Example 17**

Polyether resin

The following constituents were charged to a reaction vessel equipped with a stirrer, a thermometer and a condenser: 28.8 grams of monopentaerythritol, 187.1 grams of GE9S, 18.3 grams of n-butylacetate and 0.3550 grams of Tin (II) 2-ethylhexanoate. The mixture was heated to a temperature of about 180° C for about 5.5 hours till the GE9S was converted to an epoxy group content of about 29 mmol/kg. After cooling down the polyether had a solids content of about 95%.

**Example 18 Comparative**

Polyether resin

The following constituents were charged to a reaction vessel equipped with a stirrer, a thermometer and a condenser: 28.8 grams of monopentaerythritol, 189.4 grams of GE9H, 18.5 grams of n-butylacetate and 0.3572 grams of Tin (II) 2-ethylhexanoate. The mixture was heated to a temperature of about 180° C for about 4 hours till the GE9H was converted to an epoxy group content of about 27 mmol/kg. After cooling down the polyether had a solids content of about 95%.

**Formulation of the Clear Coats**

A clear coat is formulated with one of the polyether (from examples 16, 17, or 18, the curing agent (HDI, Desmodur N3390), the thinner (Methyl Amyl Ketone), the levelling agent (BYK-331) and the catalyst (dibutyltin dilaurate, DBTDL) according to the amounts indicated in table 13.

**Table 13:** Clear coats, formulations

| CEP-Example | Binder (ID) | Binder (g) | HDI (g) | BYK 10 wt% (g) | DBTDL 1wt% (g) | Thinner (g) |
|---|---|---|---|---|---|---|
| **CEP-16** | From Example 16 | 40.1 | 30.7 | 0.47 | 1.03 | 15.1 |
| **CEP-17** | From Example 17 | 40.0 | 33.0 | 0.48 | 1.07 | > 12.5 |
| **CEP-18** | From Example 18 | 40.0 | 32.5 | 0.48 | 1.06 | 17.7 |

**Characterization of the Clear Coats**

The clearcoat formulations (from table 13) are barcoat applied on degreased Q-panel, optionally on basecoated Q-panel. The panels are dried at room temperature after a preliminary stoving at 60°C for 30 min. Clear coats have been characterized among others by measuring the Koenig hardness development (see table 14).

**Table 14:** Clear coats, drying (curing) properties

| | CEP-16 | CEP-17 | CEP-18 | |
|---|---|---|---|---|
| **1°/** Koenig Hardness (Degreased Q panels) (sec) | | | | |
| 6 hours | 8 | 10 | 11 | |
| 24 hours | 10 | 11 | 47 | |
| 7 days | 18 | 20 | 94 | |
| **2°/** Koenig Hardness (Basecoated Q panels) (sec) | | | | |
| 6 hours | 7 | 8 | 7 | |
| 24 hours | 8 | 8 | 14 | |
| 7 days | 12 | 13 | 34 | |

**Example 19**

Preparation for Vacuum infusion of composite structures

A resin for vacuum infusion of large structures such as yacht and wind turbines was prepared by mixing 27.7 part by weight of curing agent blend and 100 part of epoxy resins blend described here:

Epoxy resins blend: 850 part by weight Epikote 828 and 150 part of glycidyl neononanoate, GE9S.

Curing Agent blend: 650 part by weight of Jeffamine D230 and 350 part by weight of Isophorone diamine (IPDA).

Jeffamine D230 is a polyoxyalkyleneamines available from Huntsman Corporation. Epikote 828 is an epoxy resin available from Momentive Specialty Chemicals

**Example 20**

Example of trowellable floor and patching compound

The ingredients presented in the table 15 below were mixed for the preparation of a trowellable flooring compound

**Table 15:** Preparation of a trowellable flooring compound

| BASE COMPONENT | Weight (parts) | Volume (parts) | Supplier |
|---|---|---|---|
| EPIKOTE 828LVEL | 63.2 | 126.3 | Momentive |
| GE9S | 11.1 | 22.3 | |
| Byk A530 | 4.8 | 13.4 | Byk Chemie |
| *Mix the additives into the EPIKOTE resin before filler addition* | | | |
| Total | 79.1 | 162.0 | |
| FILLERS | Weight (parts) | Volume (parts) | Supplier |
| Sand 1-2 mm | 582.3 | 496.4 | SCR Sibelco |
| Sand 0.2-0.6 mm | 298.4 | 254.4 | SCR Sibelco |
| Total | 880.7 | 750.8 | |
| *Disperse into the base component using a concrete mixer* | | | |
| CURING AGENT COMPONENT | Weight (parts) | Volume (parts) | Supplier |
| EPIKURE F205 | 40.2 | 87.2 | Momentive |
| Total | 40.2 | 87.2 | |
| *Mix the curing agent well with the EPIKOTE resin base and Fillers before application* | | | |

(continued)

| CURING AGENT COMPONENT | Weight (parts) | Volume (parts) | Supplier |
|---|---|---|---|
| Total formulation | 1000.0 | 1000.0 | |

**Example 21**

Formulation for a water based self-leveling flooring

The ingredients presented in the table 16 below were mixed for the preparation of a waterbased self leveling flooring system.

| CURING AGENT COMPONENT (A) | Weight (parts) | Supplier | Comment |
|---|---|---|---|
| EPIKURE 8545-W-52 (HEW = 320 g/eq) | 164.00 | Momentive | |
| EPIKURE 3253 | 4.00 | Momentive | Accelerator |
| BYK 045 | 5.00 | BYK CHEMIE | defoamer |
| Antiterra 250 | 4.00 | BYK CHEMIE | Dispersing |
| Byketol WS | 5.00 | BYK CHEMIE | Wetting agent |
| Bentone EW (3% in water) | 20.00 | Elementis | Anti-settling |
| *Mix the additive into the EPIKURE curing agents before filler addition* | | | |
| Titanium dioxide 2056 | 50.00 | KronosTitan | |
| *Disperse the pigment for 10 minutes at 2000 rpm.* | | | |
| EWO-Heavy Spar | 195.00 | Sachtleben Chemie | Barium sulphate |
| Quartz powder W8 | 98.00 | Westdeutsche Quarzwerke | |
| *Disperse fillers at 2000 rpm for 10 minutes* | | | |
| Water | 55.00 | | |
| Sand 0.1-0.4 mm | 400.00 | Euroquarz | |
| Total component A | 1000.00 | | |
| RESIN COMPONENT (B) | | | |
| EPIKOTE 828LVEL | 81.00 | Momentive | |
| GE9S | 19.00 | | |
| *Mix (B) into (A)* | | | |
| Total formulation A + B | 1081.00 | | |

**Formulation characteristics**

**Table 16:** Preparation of a waterbased self leveling flooring system

| | | |
|---|---|---|
| Fillers+Pigment / Binder ratio | 3.9 | by weight |
| PVC | 37.7 | % v/v |
| Density | 1.9 | g/ml |
| Water content | 12.5 | % m/m |

## Claims

1. A composition of $\alpha,\alpha$-branched alkane carboxylic glycidyl esters from butene oligomers, **characterized in that** the glycidyl ester mixture is based on neononanoic (C9) acid mixture where the sum of the concentration of the blocked isomers and the concentration of the highly branched isomers is maximum 55%, preferably below 40%, and most preferably below 30% weight on total composition, and the glycidyl ester mixture is comprising 2,2-dimethyl heptanoic

acid glycidyl ester and 2-methyl 2-ethyl hexanoic acid glycidyl ester and 2-methyl 2-ethyl 3-methyl pentanoic acid glycidyl esters.

2. The composition of claim 1 **characterized in that** the glycidyl ester mixture is comprising 2,2-dimethyl heptanoic acid glycidyl ester in 4 to 10 weight% and 2-methyl 2-ethyl hexanoic acid glycidyl ester in 40 to 70 weight% and 2-methyl 2-ethyl 3-methyl pentanoic acid glycidyl esters (sum of stereoisomers) in 10 to 25 weight% on total composition.

3. The composition of claim 1 **characterized in that** the glycidyl ester mixture is comprising 2,2-dimethyl heptanoic acid glycidyl ester in 5 to 10 weight% and 2-methyl 2-ethyl hexanoic acid glycidyl ester in 45 to 65 weight% and 2-methyl 2-ethyl 3-methyl pentanoic acid glycidyl esters (sum of stereoisomers) in 12 to 22 weight% on total composition.

4. The composition of claim 1 **characterized in that** the glycidyl ester mixture is comprising 2,2-dimethyl heptanoic acid glycidyl ester in 6 to 9 weight% and 2-methyl 2-ethyl hexanoic acid glycidyl ester in 47 to 61 weight% and 2-methyl 2-ethyl 3-methyl pentanoic acid glycidyl esters (sum of stereoisomers) in 14 to 21 weight% on total composition.

5. The use of the glycidyl esters compositions, of any previous claims, as reactive diluent or as momomer in binder compositions for paints and adhesives.

6. A polyester polyol resin comprising a glycidyl ester of any previous claims.

7. An acrylic polyol resin comprising a glycidyl ester of any previous claims.

8. A polyether polyol resin comprising a glycidyl ester of any previous claims.

9. An epoxy resin formulation comprising a glycidyl ester of any previous claims.

**Patentansprüche**

1. Zusammensetzung von α,α-verzweigten Alkancarbonsäure-Glycidylestern aus Butenoligomeren, **dadurch gekennzeichnet, dass** die Glycidylestermischung auf einem Neononansäuregemisch (C9) basiert, wobei die Summe der Konzentration der blockierten Isomere und der Konzentration der hoch verzweigten Isomere maximal 55 %, vorzugsweise unter 40 % und am meisten bevorzugt unter 30 %, bezogen auf die Gesamtzusammensetzung, beträgt, und das Glycidylestergemisch 2,2-Dimethylheptansäureglycidylester und 2-Methyl-2-ethylhexansäureglycidylester und 2-Methyl-2-ethyl-3-methylpentansäureglycidylester umfasst.

2. Zusammensetzung nach Anspruch 1, **dadurch gekennzeichnet, dass** die Glycidylestermischung 4 bis 10 Gew.-% 2,2-Dimethylheptansäureglycidylester und 40 bis 70 Gew.-% 2-Methyl-2-ethylhexansäureglycidylester und 10 bis 25 Gew.-% 2-Methyl-2-ethyl-3-methylpentansäureglycidylester (Summe der Stereoisomere) bezogen auf die Gesamtzusammensetzung enthält.

3. Zusammensetzung nach Anspruch 1, **dadurch gekennzeichnet, dass** die Glycidylestermischung 5 bis 10 Gew.-% 2,2-Dimethylheptansäureglycidylester und 45 bis 65 Gew.-% 2-Methyl-2-ethylhexansäureglycidylester und 12 bis 22 Gew.-% 2-Methyl-2-ethyl-3-methylpentansäureglycidylester (Summe der Stereoisomere) der Gesamtzusammensetzung enthält.

4. Zusammensetzung nach Anspruch 1, **dadurch gekennzeichnet, dass** die Glycidylestermischung 6 bis 9 Gew.-% 2,2-Dimethylheptansäureglycidylester und 47 bis 61 Gew.-% 2-Methyl-2-ethylhexansäureglycidylester und 14 bis 21 Gew.-% 2-Methyl-2-ethyl-3-methylpentansäureglycidylester (Summe der Stereoisomere) der Gesamtzusammensetzung enthält.

5. Verwendung der Glycidylester-Zusammensetzungen nach einem der vorhergehenden Ansprüche als reaktives Verdünnungsmittel oder als Monomer in Bindemittelzusammensetzungen für Farben und Klebstoffe.

6. Polyesterpolyolharz, umfassend einen Glycidylester nach einem der vorhergehenden Ansprüche.

7. Acrylpolyolharz, umfassend einen Glycidylester nach einem der vorhergehenden Ansprüche.

8. Polyetherpolyolharz, umfassend einen Glycidylester nach einem der vorhergehenden Ansprüche.

9. Epoxidharzformulierung, umfassend einen Glycidylester nach einem der vorhergehenden Ansprüche.

## Revendications

1. Composition d'esters glycidyliques d'alcane carboxyliques a, α-ramifiés à partir d'oligomères de butène, **caractérisée en ce que** le mélange d'ester glycidylique est basé sur un mélange d'acide néononanoïque (C9) où la somme des concentrations des isomères bloqués et fortement ramifiés est maximale de 55%, de préférence inférieure à 40% et de préférence inférieure à 30% en poids de la composition totale et que le mélange d'ester glycidylique comprend l'ester glycidylique d'acide 2,2-diméthyl heptanoïque et l'ester glycidylique d'acide 2-méthyl 2-éthyl hexanoïque et le glycidyl ester d'acide 2-méthyl 2-éthyl 3-méthyl pentanoïque.

2. Composition selon la revendication 1, **caractérisée en ce que** le mélange d'ester glycidylique comprend l'ester glycidylique d'acide 2,2-diméthyl-heptanoïque dans 4 à 10% en poids et l'ester glycidylique d'acide 2-méthyl 2-éthyl hexanoïque dans 40 à 70% en poids et l'ester glycidylique de l'acide 2-méthyl 2-éthyl 3-méthyl pentanoïque (somme des stéréoisomères) en une quantité de 10 à 25% en poids de la composition totale.

3. Composition selon la revendication 1, **caractérisée en ce que** le mélange d'ester glycidylique comprend l'ester glycidylique d'acide 2,2-diméthyl-heptanoïque dans 5 à 10% en poids et l'ester glycidylique d'acide 2-méthyl 2-éthyl hexanoïque dans 45 à 65% en poids et l'ester glycidylique de l'acide 2-méthyl 2-éthyl 3-méthyl pentanoïque (somme des stéréoisomères) en une quantité de 12 à 22% en poids de la composition totale.

4. Composition selon la revendication 1, **caractérisée en ce que** le mélange d'ester glycidylique comprend l'ester glycidylique d'acide 2,2-diméthyl-heptanoïque dans 6 à 9% en poids et l'ester glycidylique d'acide 2-méthyl 2-éthyl hexanoïque dans 47 à 61% en poids et l'ester glycidylique de l'acide 2-méthyl 2-éthyl 3-méthyl pentanoïque (somme des stéréoisomères) en une quantité de 14 à 21% en poids de la composition totale.

5. Utilisation des compositions d'esters glycidylique, selon l'une quelconque des revendications précédentes, en tant que diluant réactif ou en tant que momomère dans des compositions de liant pour peintures et adhésifs.

6. Une résine de polyester polyol comprenant un ester glycidylique selon l'une quelconque des revendications précédentes.

7. Une résine de polyol acrylique comprenant un ester glycidylique selon l'une quelconque des revendications précédentes.

8. Une résine de polyéther polyol comprenant un ester glycidylique selon l'une quelconque des revendications précédentes.

9. Une formulation de résine époxy comprenant un ester glycidylique selon l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2831877 A
- US 2876241 A
- US 3053869 A
- US 2967873 A
- US 3061621 A
- EP 1033360 A1
- EP 2010003334 W
- US 6433217 B
- WO 9951659 A
- EP 1283226 A1
- JP 4314797 A
- WO 9633245 A
- JP 60032803 A
- US 6342615 B1